# EUROPEAN PATENT APPLICATION

(11) **EP 2 030 618 A2**
(43) Date of publication of application: **04.03.2009**
(21) Application number: 08169239.4
(22) Date of filing: 13.01.2003
(51) Int. Cl.: A61K 31/427, A61K 31/513, A61K 31/7068, A61P 35/00

(54) **Combinations comprising epothilones and anti-metabolites**

(30) Priority: 14.01.2002 US 348622 P; 04.10.2002 US 416173 P
(62) Divisional of application: 03729249.7
(71) Applicant: NOVARTIS AG, 4002 Basel (CH); Novartis Pharma GmbH, 1235 Vienna (AT)
(72) Inventor: Hohneker, John Arthur, Morristown, NC 07960 (US); Mcsheehy, Paul M.J., 79540 Loerrach-Stetten (DE); Rotthermel, John David, Sunset Beach, NC 28468 (US)
(74) Representative: Roth, Peter Richard

(57) **Abstract**

Disclosed is a combination which comprises (a) an antineoplastic antimetabolite and (b) an epothilone derivative of formula 1 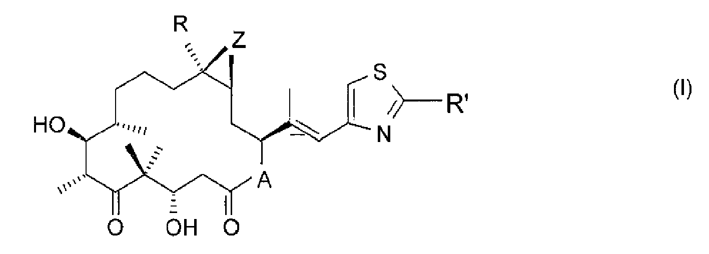 wherein A represents O or NR_{N}, wherein R_{N} is hydrogen or lower alkyl, R is hydrogen or lower alkyl, R' is methyl, methoxy, ethoxy, amino, methylamino, dimethylamino, aminomethyl or methylthio, and Z is O or a bond, and optionally at least one pharmaceutically acceptable carrier and/or, optionally, a standard anti-diarrheal, for simultaneous, separate or sequential use, in particular for the treatment of a proliferative disease; a pharmaceutical composition comprising such a combination; the use of such a combination for the preparation of a medicament for the treatment of a proliferative disease; a commercial package or product comprising such a combination as a combined preparation for simultaneous, separate or sequential use; and to a method of treatment of a warm-blooded animal.

## Description

The invention relates to a combination which comprises (a) an antineoplastic antimetabolite and (b) an epothilone derivative of formula I and optionally at least one pharmaceutically acceptable carrier for simultaneous, separate or sequential use, in particular for the treatment of a proliferative disease, especially a solid tumor disease; a pharmaceutical composition comprising such a combination; the use of such a combination for the preparation of a medicament for the treatment of a proliferative disease; a commercial package or product comprising such a combination as a combined preparation for simultaneous, separate or sequential use; and to a method of treatment of a warm-blooded animal, especially a human.

For more than three decades 5-fluorouracil (5-FU) has been the mainstay of chemotherapeutic agents for colorectal cancer. Oral prodrugs of 5-FU such as capecitabine (XELODA^{™}) and tegafur (FTORAFUR^{™}) recently have been developed, which are more selectively active on tumor cells. The oral prodrugs may be more convenient and better tolerated than the schedule of 5-FU. However, these oral agents have the same profile of tolerance as the 5-FU given intraveniously and diarrhea and hand-foot syndrome continue to be problems (see, e.g., Rougier P, Mitry E. Current Treatment Options for Advanced Colorectal Cancer, Seminars in Oncology 27 (5), 2000, 30-33).

The microtubule-stabilizing effect of epothilones was first described by Bollag et al., Cancer Research 55, 1995, 2325-33. A suitable treatment schedule of different types of tumors, especially tumors which are refractory to the treatment by other chemotherapeutics, in particular refractory to the treatment by taxanes, like TAXOL^{™}, is described in WO 99/43320.

The present invention pertains to a combination, such as a combined preparation or a pharmaceutical composition, which comprises (a) an antineoplastic antimetabolite and (b) an epothilone derivative of formula I 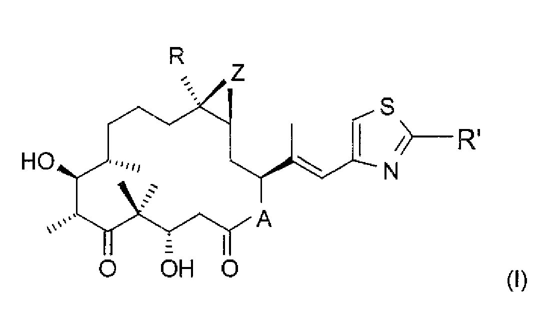 wherein A represents O or NR_{N}, wherein R_{N} is hydrogen or lower alkyl, R is hydrogen or lower alkyl, R' is methyl, methoxy, ethoxy, amino, methylamino, dimethylamino, aminomethyl or methylthio, and Z is O or a bond,
in which the active ingredients (a) and (b) are present in each case in free form or in the form of a pharmaceutically acceptable salt and, optionally, at least one pharmaceutically acceptable carrier and/or, optionally, a standard anti-diarrheal; for simultaneous, separate or sequential use, in particular for the treatment of a proliferative disease, especially a solid tumor disease.

Unless stated otherwise, in the present disclosure organic radicals and compounds designated "lower" contain not more than 7, preferably not more than 4, carbon atoms.

The term "a combined preparation", as used herein defines especially a "kit of parts" in the sense that the combination partners (a) and (b) as defined above can be dosed independently or by use of different fixed combinations with distinguished amounts of the combination partners (a) and (b), i.e., simultaneously or at different time points. The parts of the kit of parts can then, e.g., be administered simultaneously or chronologically staggered, that is at different time points and with equal or different time intervals for any part of the kit of parts. Very preferably, the time intervals are chosen such that the effect on the treated disease in the combined use of the parts is larger than the effect which would be obtained by use of only any one of the combination partners (a) and (b). The ratio of the total amounts of the combination partner (a) to the combination partner (b) to be administered in the combined preparation can be varied, e.g. in order to cope with the needs of a patient subpopulation to be treated or the needs of the single patient which different needs can be due to age, sex, body weight, etc. of the patients. Preferably, there is at least one beneficial effect, e.g., a mutual enhancing of the effect of the combination partners (a) and (b), in particular a synergism, e.g. a more than additive effect, additional advantageous effects, less side effects, a combined therapeutical effect in a non-effective dosage of one or both of the combination partners (a) and (b), and very preferably a strong synergism of the combination partners (a) and (b).

The term "treatment" comprises the administration of the combination partners to a warm-blooded animal in need of such treatment with the aim to effect a delay of progression of a disease.

The term "a solid tumor disease" especially means cancer of the colon or the rectum, caecum cancer and generally cancer of the GI tract, ovarian cancer, cervix cancer, lung cancer, e.g. small-cell lung cancer and non-small-cell lung cancer, head and neck cancer, breast cancer, pancreas cancer, renal cancer (in particular cancer of the kidney or the adrenal), skin cancer, bladder cancer, cancer of the prostate, the thyroid, the vulva, adenocarcinoma or Kaposi's sarcoma, and metastases thereof. The combinations disclosed herein are also useful for the treatment of leukemia.

The term "antineoplastic antimetabolites" includes, but is not limited to, 5-fluorouracil, tegafur, capecitabine, cladribine, cytarabine, fludarabine phosphate, fluorouridine, gemcitabine, 6-mercaptopurine, hydroxyurea, methotrexate, edatrexate and salts of such compounds, and furthermore ZD 1694 (RALTITREXED^{™}), LY231514 (ALIMTA^{™}), LY264618 (LOMOTREXOL^{™}) and OGT719.

5-Fluorouracil can be prepared, e.g., as described in US 2,802,005. It can be employed in the present invention as marketed, e.g., under the trademark EFUDEX™, FLURACIL™ or FLUROBLASTIN™. Tegafur can be employed especially in the form of a composition as disclosed in US 5,116,600 and US 5,525,603. Furthermore, tegafur can be administered, e.g., in the form as it is marketed under the trademarks FTORAFUR™, LAMAR™ or NEBEREK^{™}. Capecitabine can be administered, e.g., in the form as disclosed in US 5,472,949 or in the form as it is marketed, e.g., under the trademark XELODA™. Cladribine can be prepared, e.g., as disclosed in US 4,760,135. It can be administered, e.g., in the form as it is marketed under the trademarks LEUSTATIN™ or LEUSTAT™. Cytarabine can, e.g., be prepared as disclosed in US 3,116,282 or by Hessler in J. Org. Chem. 41 (1970) 1828. It can be administered, e.g., in the form as it is marketed under the trademarks ARA-C™, CYTOSAR™ or UDICIL™. A suitable salt of such compound is cytarabine ocfosfate (STARASID^{™}) which can be prepared as described in US 4,812,560. Fludarabine phosphate can be prepared as described in US 4,357,324. It can be applied as marketed under the trademark FLUDARA™. Gemcitabine can be administered, e.g., in accordance with the disclosure of US 5,464,826 or in the form as it is marketed, e.g., as gemcitabine hydrochloride under the trademark GEMZAR™. 6-Mercaptopurine (6-purinethiol) can, e.g., be prepared as disclosed in US 2,933,498. It can be employed as marketed, e.g., under the trademark LEUKERIN™ or PURINETHOL™. Hydroxyurea can, e.g., be prepared as disclosed in US 2,705,727. Methotrexate can be employed as marketed, e.g., under the trademark FOLEX^{™} or MTX^{™}. Edatrexate can, e.g., be prepared as disclosed in US 4,369,319.

The term "standard anti-diarrheal" as used herein include, but is not limited to, natural opiods, such as tincture of opium, paregoric, and codeine, synthetic opoids, such as diphenoxylate, difenoxin and loperamide, bismuth subsalicylate, octreotide, motilin antagonists and traditional antidiarrheal remedies, such as kaolin, pectin, berberine and muscarinic agents. The antidiarrheal agent is administered as a preventative measure throughout the treatment cycle or as needed as soon as diarrhea occurs. The antidiarrheal agent is administered to prevent, control or eliminate diarrhea that is sometimes associated with the administration of epothilones, especially epothilone B.

The structure of the active agents identified by code nos., generic or trade names may be taken from the actual edition of the standard compendium "The Merck Index" or from databases, e.g. Patents International (e.g. IMS World Publications). The corresponding content thereof is hereby incorporated by reference.

A compound of formula I wherein A represents O, R is hydrogen, R' is methyl and Z is O is known as epothilone A; a compound of formula I wherein A represents O, R is methyl, R' is methyl and Z is O is known as epothilone B; a compound of formula I wherein A represents O, R is hydrogen, R' is methyl and Z is a bond is known as epothilone C; a compound of formula I wherein A represents O, R is methyl, R' is methyl and Z is a bond is known as epothilone D.

The compounds used as combination partners (a) and (b) disclosed herein can be prepared and administered as described in the cited documents, respectively, if not mentioned otherwise.

Epothilone derivatives of formula I wherein A represents O or NR_{N}, wherein R_{N} is hydrogen or lower alkyl, R is hydrogen or lower alkyl, R' is methyl and Z is O or a bond, and methods for the preparation of such epothilone derivatives are in particular generically and specifically disclosed in the patents and patent applications WO 93/10121, US 6,194,181, WO 98/25929, WO 98/08849, WO 99/43653, WO 98/22461 and WO 00/31247 in each case in particular in the compound claims and the final products of the working examples. Tthe subject-matter of the final products, the pharmaceutical preparations and the claims is hereby incorporated into the present application by reference to these publications. Comprised are likewise the corresponding stereoisomers as well as the corresponding crystal modifications, e.g. solvates and polymorphs, which are disclosed therein.

Epothilone derivatives of formula I, especially epothilone B, can be administered as part of pharmaceutical compositions which are disclosed in WO 99/39694. Epothilone B can be stored in individual 10 ml glass vials each containing 4 mL of the clear, colorless drug concentrate formulated in polyethylene glycol. The concentrate must be diluted in 0.9 % aqueous sodium chloride solution before use.

Epothilone derivatives of formula I wherein A represents O or NR_{N}, wherein R_{N} is hydrogen or lower alkyl, R is hydrogen or lower alkyl, R' is methoxy, ethoxy, amino, methylamino, dimethylamino, aminomethyl or methylthio, and Z is O or a bond, and methods for the preparation and administration of such epothilone derivatives are in particular generically and specifically disclosed in the patent application WO99/67252, which is hereby incorporated by reference. Comprised are likewise the corresponding stereoisomers as well as the corresponding crystal modifications, e.g. solvates and polymorphs, which are disclosed therein.

The transformation of epothilone B to the corresponding lactam is disclosed in Scheme 21 (page 31, 32) and Example 3 of WO 99/02514 (pages 48 - 50). The transformation of a compound of formula I which is different from epothilone B into the corresponding lactam can be accomplished analogously. Corresponding epothilone derivatives of formula I
wherein R_{N} is lower alkyl can be prepared by methods known in the art such as a reductive alkylation reaction starting from the epothilone derivative wherein R_{N} is hydrogen.

A compound of formula I, wherein A represents O, R is methyl, R' is aminomethyl and Z is O can be prepared as described in Examples 13 to 16 of WO01/72721 starting with a compound of formula I, wherein A represents O, R and R' are both methyl and Z is O.

It will be understood that references to the combination partners (a) and (b) are meant to also include the pharmaceutically acceptable-salts. If these combination partners (a) and (b) have, for example, at least one basic center, they can form acid addition salts, e.g. succinates. Corresponding acid addition salts can also be formed having, if desired, an additionally present basic center. The combination partners (a) and (b) having an acid group (for example COOH) can also form salts with bases. The combination partner (a) or (b) or a pharmaceutically acceptable salt thereof may also be used in form of a hydrate or include other solvents used for crystallization.

A combination which comprises (a) an antineoplastic antimetabolite and (b) an epothilone derivative of formula I wherein A represents O or NR_{N}, wherein R_{N} is hydrogen or lower alkyl, R is hydrogen or lower alkyl, R' is methyl, methoxy, ethoxy, amino, methylamino, dimethylamino, aminomethyl or methylthio, and Z is O or a bond, in which the-active ingredients are present in each case in free form or in the form of a pharmaceutically acceptable salt and optionally at least one pharmaceutically acceptable carrier, will be referred to hereinafter as a COMBINATION OF THE INVENTION.

The COMBINATIONS OF THE INVENTION inhibit the growth of solid tumors, but also liquid tumors. Furthermore, the COMBINATIONS OF THE INVENTION exhibit beneficial effects in the treatment of diseases associated with deregulated angiogenesis. In one preferred embodiment of the invention, the proliferative disease to be treated with a COMBINATION OF THE INVENTION is colorectal cancer or breast cancer.

The nature of proliferative diseases like solid tumor diseases is multifactorial. Under certain circumstances, drugs with different mechanisms of action may be combined. However, just considering any combination of drugs having different mode of action does not necessarily lead to combinations with advantageous effects.

All the more surprising is the experimental finding that *in vivo* the administration of a COMBINATION OF THE INVENTION compared to a monotherapy applying only one of the pharmaceutically active ingredients used in the COMBINATION OF THE INVENTION results not only in a more beneficial, especially synergistic, e.g. anti-proliferative effect, e.g. with regard to the delay of progression of a proliferative disease or with regard to a change in tumor volume, but also in further surprising beneficial effects, e.g. less side-effects and a decreased mortality and morbidity. Furthermore, depending on the tumor type and the particular combination used a decrease of the tumor volume can be obtained when using a COMBINATION OF THE INVENTION in cases in which by monotherapy no decrease of the tumor volume can be achieved. The COMBINATIONS OF THE INVENTION are also suitable to prevent the metastatic spread of tumors and the growth or development of micrometastases. The COMBINATIONS OF THE INVENTION are in particular suitable for the treatment of patients with advanced cancer who have failed standard systemic therapy. This includes patients having tumor types showing resistance to monotherapy, especially monotherapy with an antineoplastic antimetabolites or a taxane like TAXOL^{™}, or showing resistance to combinations different from those disclosed herein.

A further benefit is that lower doses of the active ingredients of the COMBINATION OF THE INVENTION can be used, for example, that the dosages need not only often be smaller but are also applied less frequently, or can be used in order to diminish the incidence of side-effects like, e.g., diarrhea or hand-foot syndrome observed with one of the combination partners alone. This is in accordance with the desires and requirements of the patients to be treated.

It can be shown by established test models that a COMBINATION OF THE INVENTION results in the beneficial effects described herein-before. The person skilled in the pertinent art is fully enabled to select a relevant test model to prove such beneficial effects. The pharmacological activity of a COMBINATION OF THE INVENTION may, for example, be demonstrated in a clinical study or in a test procedure as essentially described hereinafter.

Suitable clinical studies are in particular randomized, double-blind, placebo-controlled, parallel studies in cancer patients with late stage disease. Such studies are, in particular, suitable to compare the effects of a monotherapy using the active ingredients and a therapy using a COMBINATION OF THE INVENTION, and to prove in particular the synergism of the active ingredients of the COMBINATIONS OF THE INVENTION. Tumor response can be classified as complete response, partial response or stable disease as defined by the RECIST solid tumor response criteria (summarized below) and can be assessed by the appropriate radiographic or physical examination at the end of every two cycles and at the end of the study. The radiologic evaluation of tumors in regular time periods, e.g. every 4, 6, 8 or 10 weeks, is a suitable approach to determine the effect of the COMBINATION OF THE INVENTION. The primary endpoints in such studies can be the effect on pain scores, analgesic use, performance status, Quality of Life scores or time to progression of the disease. In a suitable study design, patients are, for example, randomized in a double-blind fashion receiving per treatment cycle of four weeks a fixed dosage ranging from about 500 to 1500 mg/m² twice daily, in particular 1250 mg/m² twice daily, of XELODA^{™} for two weeks followed by one or two weeks without such antimetabolite or a corresponding placebo in addition to treatment cycles employing a compound of formula I, e.g. epothilone B, wherein each cycle consists of 0.5, 1.0, 1.5, 2.0 or 2.5 mg/m² epothilone B administered as a 5 minute bolus injection once a week for three weeks followed by one week of rest. Alternatively, the compound of formula I can be administered once every three weeks. The minimum duration of such a study should be about 8 weeks.

The term "complete response" as used herein means in particular to the resolution of all measurable or evaluable disease.

The term "partial response" as used herein means in particular a greater than or equal to 50 % reduction in measurable or evaluable disease in the absence of progression in any particular disease site.

The term "stable disease" as used herein means in particular a less than 50 % decrease or less than 25 % increase in measurable or evaluable disease.

It is one objective of this invention to provide a pharmaceutical composition comprising a quantity, which is jointly therapeutically effective against a proliferative disease comprising the COMBINATION OF THE INVENTION. In this composition, the combination partners (a) and (b) can be administered together, one after the other or separately in one combined unit dosage form or in two separate unit dosage forms. The unit dosage form may also be a fixed combination.

All patients receiving the COMBINATION OF THE INVENTION, in particular a combination comprising epothilone B, experiencing diarrhea should be treated as soon as possible, e.g., according to the following algorithm or a treatment algorithm in accordance with local guidelines.

**Table 1**

| Diarrhea Grade | Grade 1 | Grade 2 | Grade 3 or 4 |
|---|---|---|---|
| Standard Loperamide¹ | 24 hours | | |
| High-dose Loperamide² | 24-48 hours | 12-24 hours | |
| Sandostatin 150 ug SC tid | 24-48 hours | 12-24 hours | |
| Sandostatin 250 ug SC tid | 24-48 hours | 12-24 hours | 12 hours |
| Sandostatin 500 ug SC tid | until resolved | 12-24 hours | 12-24 hours |
| Sandostatin 750 ug SC tid | | 12-24 hours | **↓** |
| Sandostatin 1000 ug SC tid | | until resolved | 12-24 hours |
| High dose opiates (if appropriate), Or Sandostatin 1000 ug IV tid | | | until resolved |

| | | | |
|---|---|---|---|
| ¹- Standard loperamide: 4mg to start, then 2mg every four hours or after every loose stool, as per OTC package insert. ²- High-dose loperamide: 4 mg to start, then 2 mg every two hours, as per Irinotecan package insert | | | |

Patients should be treated at the appropriate step for the minimal time indicated; if the diarrhea does not resolve, treatment at the next higher step may be initiated at any time, but should not be delayed any longer than the maximal time indicated. If the diarrhea increases in grade at any time, treatment at the next higher step for the new grade should be in initiated. All therapy should continue until diarrhea has resolved for at least 12 hours; if the diarrhea decreases in grade but does not resolve, ongoing therapy may be continued or reduced at the investigator's discretion. If diarrhea reoccurs following a break in treatment, therapy should be re-initiated at the next higher step if the grade is the same or two steps up if the grade has increased.

The pharmaceutical compositions according to the invention can be prepared in a manner known per se and are those suitable for enteral, such as oral or rectal, and parenteral administration to mammals (warm-blooded animals), including man, comprising a therapeutically effective amount of at least one pharmacologically active combination partner alone or in combination with one or more pharmaceutically acceptable carries, especially suitable for enteral or parenteral application. In one embodiment of the invention, one or more of the active ingredients are administered intraveniously.

The novel pharmaceutical composition contain, for example, from about 10 % to about 100 %, preferably from about 20 % to about 60 %, of the active ingredients. Pharmaceutical preparations for the combination therapy for enteral or parenteral administration are, for example, those in unit dosage forms, such as sugar-coated tablets, tablets, capsules or suppositories, and furthermore ampoules. If not indicated otherwise, these are prepared in a manner known per se, for example by means of conventional mixing, granulating, sugar-coating, dissolving or lyophilizing processes. It will be appreciated that the unit content of a combination partner contained in an individual dose of each dosage form need not in itself constitute an effective amount since the necessary effective amount can be reached by administration of a plurality of dosage units.

In particular, a therapeutically effective amount of each of the combination partners of the COMBINATION OF THE INVENTION may be administered simultaneously or sequentially and in any order, and the components may be administered separately or as a fixed combination. For example, the method of delay of progression or treatment of a proliferative disease according to the invention may comprise (i) administration of the first combination partner in free or pharmaceutically acceptable salt form and (ii) adminstration of the second combination partner in free or pharmaceutically acceptable salt form, simultaneously or sequentially in any order, in jointly therapeutically effective amounts, preferably in synergistically effective amounts, e.g. in daily dosages corresponding to the amounts described herein. The individual combination partners of the COMBINATION OF THE INVENTION can be administered separately at different times during the course of therapy or concurrently in divided or single combination forms. Furthermore, the term administering also encompasses the use of a pro-drug of a combination partner that convert *in vivo* to the combination partner as such. The instant invention is therefore to be understood as embracing all such regimes of simultaneous or alternating treatment and the term "administering" is to be interpreted accordingly.

The effective dosage of each of the combination partners employed in the COMBINATION OF THE INVENTION may vary depending on the particular compound or pharmaceutical composition employed, the mode of administration, the condition being treated, the severity of the condition being treated. Thus, the dosage regimen the COMBINATION OF THE INVENTION is selected in accordance with a variety of factors including the route of administration and the renal and hepatic function of the patient. A physician, clinician or veterinarian of ordinary skill can readily determine and prescribe the effective amount of the single active ingredients required to prevent, counter or arrest the progress of the condition. Optimal precision in achieving concentration of the active ingredients within the range that yields efficacy without toxicity requires a regimen based on the kinetics of the active ingredients' availability to target sites. This involves a consideration of the distribution, equilibrium, and elimination of the active ingredients.

When the combination partners employed in the COMBINATION OF THE INVENTION are applied in the form as marketed as single drugs, their dosage and mode of administration can take place in accordance with the information provided on the packet leaflet of the respective marketed drug in order to result in the beneficial effect described herein, if not mentioned herein otherwise.

In partcular, if the the warm-blooded animal is a human, the dosage of a compound of formula I is preferably in the range of about 0.25 to 75, preferably 0.5 to 50, e.g. 2.5, mg/m² once weekly for two to four, e.g. three, weeks, followed by 6 to 8 days off in the case of an adult patient.

5-Fluorouracil may be administered to a human in a dosage range varying from about 50 to 1000 mg/m²day, e.g. 500 mg/m²day.

Capecitabine may be administered orally to a human in a dosage range varying from about 500 to 1500 mg/m²day. The compound can, e.g., be administered in the form of commercially available tablets containing 150 or 500 mg of capecitabine. In a preferred embodiment of the present invention, the dose of capecitabine is calculated according to the scheme provided in Table 2:

**Table 2**

| Dose Level 1250 mg/m² of Capecitabine twice a day | | Number of Tablets to be Taken at Each Dose (Morning and Evening) | |
|---|---|---|---|
| Surface Area (m²) | Total Daily* Dose (mg) | 150 mg | 500 mg |
| ≤ 1.25 | 3000 | 0 | 3 |
| 1.25-1.37 | 3300 | 1 | 3 |
| 1.38-1.51 | 3600 | 2 | 3 |
| 1.52-1.65 | 4000 | 0 | 4 |
| 1.56-1.77 | 4300 | 1 | 4 |
| 1.78-1.91 | 4600 | 2 | 4 |
| 1.92-2.05 | 5000 | 0 | 5 |
| 2.06-2.17 | 5300 | 1 | 5 |
| ≥ 2.18 | 5600 | 2 | 5 |

| | | | |
|---|---|---|---|
| *Total Daily Dose divided by 2 to allow equal morning and evening doses | | | |

Gemcitabine hydrochloride may be administered to a human in a dosage range varying from 10 to about 1000 mg/week or, preferably, 800 mg/m² weekly as a half hour i.v. infusion.

Methotrexate may be administered to a human in a dosage range varying from about 5 to 500 mg/m²day.

ZD 1694 (RALTITREXED^{™}) can be administered to a human in a dosage range varying from about 2.0 to 4.0 mg/m², e.g., 3.5 mg/m², every 3 weeks as a 15 minute infusion.

In the compound of formula I preferably A represents O. R is hydrogen or, preferably, lower alkyl, e.g. ethyl or, most preferably, methyl. Z is preferably O or a bond, more preferably O.

In one preferred embodiment of the invention, the antineoplastic antimetabolite is selected from 5-fluorouracil, tegafur, capecitabine, cladribine, cytarabine, fludarabine phosphate, fluorouridine, gemcitabine, 6-mercaptopurine, hydroxyurea, methotrexate and edatrexate. More preferably, it is selected from 5-fluorouracil, tegafur, gemcitabine and capecitabine. Most preferably, the antineoplastic antimetabolite is selected from gemcitabine and capecitabine.

The COMBINATION OF THE INVENTION can be a combined preparation or a pharmaceutical composition.

Moreover, the present invention relates to a method of treating a warm-blooded animal having a proliferative disease comprising administering to the animal a COMBINATION OF THE INVENTION in a quantity which is jointly therapeutically effective against a proliferative disease and in which the combination partners can also be present in the form of their pharmaceutically acceptable salts. In one embodiment of the invention, in such method the COMBINATION OF THE INVENTION is co-administered with folinic acid and/or an anti-diarrheal agent. Furthermore, the treatment can comprise surgery, radiotherapy, cryotherapy and immunotherapy.

Furthermore, the present invention pertains to the use of a COMBINATION OF THE INVENTION for the treatment of a proliferative disease and for the preparation of a medicament for the treatment of a proliferative disease.

Additionally, the present invention pertains to the use of an antineoplastic antimetabolite in combination with an epothilone derivative of formula I wherein A represents O or NR_{N}, wherein R_{N} is hydrogen or lower alkyl, R is hydrogen or lower alkyl, R' is methyl, methoxy, ethoxy, amino, methylamino, dimethylamino, aminomethyl or methylthio, and Z is O or a bond, for the preparation of a medicament for the treatment of a proliferative disease.

Moreover, the present invention provides a commercial package comprising as active ingredients COMBINATION OF THE INVENTION, together with instructions for simultaneous, separate or sequential use thereof in the treatment of a proliferative disease.

### EXAMPLE 1

A human patient having advanced renal cancer receives 0.5 mg/m² of epothilone B as a five minutes bolus infusion weekly for 3 weeks followed by one week off. Starting in the second week of the epothilone treatment and at least two hours after said treatment, capecitabine is applied orally to said patient twice daily at a dosage of 1250 mg/m² for two weeks followed by one week off. The whole treatment cycle is applied to the patient several times. Stable disease is observed for about 8 months.

### EXAMPLE 2

A human patient having advanced cancer receives 2.0 mg/m² of epothilone B as a five minutes bolus infusion weekly for 3 weeks followed by one week off. Starting in the second week of the epothilone treatment at least two hours after said treatment, capecitabine is applied orally to said patient twice daily at a dosages of 1250 mg/m² for two weeks followed by one week off. The whole treatment cycle is applied several times.

### EXAMPLE 3

A human patient having advanced cancer receives 2.5 mg/m² of epothilone B as a five minutes bolus infusion weekly for 3 weeks followed by one week off. Starting in the second week of the epothilone treatment at least two hours after said treatment, capecitabine is applied orally to said patient twice daily at a dosage of 1250 mg/m² for two weeks followed by one week off. The whole treatment cycle is applied several times.

### EXAMPLE 4

A human patient having advanced cancer receives 3.0 mg/m² of epothilone B as a five minutes bolus infusion weekly for 2 weeks followed by one week off. At least two hours after said treatment, capecitabine is applied orally to said patient twice daily at a dosage of 1250 mg/m² for two weeks followed by one week off. The whole treatment cycle is applied several times.

### EXAMPLE 5

A human patient having advanced colon cancer receives 0.5 mg/m² of epothilone B as a five minutes bolus infusion weekly for 3 weeks followed by one week off. Immediately following said treatment, gemcitabine hydrochloride is applied orally to said patient twice daily at a dosage of 800 mg/m². The whole treatment cycle is applied to the patient several times. Stable disease is observed for about 3 ½ months.

### EXAMPLE 6

A human patient having advanced cancer receives 1.0 mg/m² of epothilone B as a five minutes bolus infusion weekly for 3 weeks followed by one week off. Immediately following said treatment, gemcitabine hydrochloride is applied orally to said patient twice daily at a dosage of 800 mg/m². The whole treatment cycle is applied several times.

### EXAMPLE 7

A human patient having advanced cancer receives 2.0 mg/m² of epothilone B as a five minutes bolus infusion weekly for 3 weeks followed by one week off. Immediately following said treatment, gemcitabine hydrochloride is applied orally to said patient twice daily at a dosage of 800 mg/m². The whole treatment cycle is applied several times.

### EXAM PLE 8

A human patient having advanced cancer receives 2.5 mg/m² of epothilone B as a five minutes bolus infusion weekly for 2 weeks followed by one week off. Immediately following said treatment, gemcitabine hydrochloride is applied orally to said patient twice daily at a dosage of 800 mg/m². The whole treatment cycle is applied several times.

## Claims

1. A combination which comprises (a) an antineoplastic antimetabolite and (b) an epothilone derivative of formula I 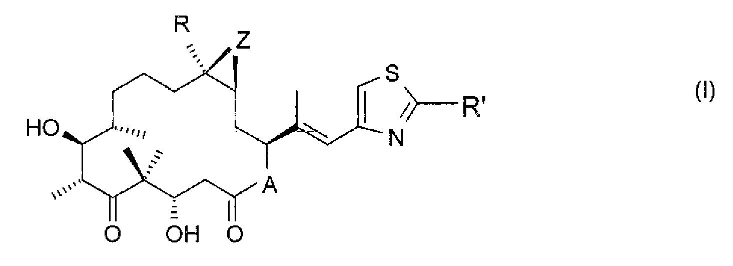 wherein A represents O or NR_{N}, wherein R_{N} is hydrogen or lower alkyl, R is hydrogen or lower alkyl, R' is methyl, methoxy, ethoxy, amino, methylamino, dimethylamino, aminomethyl or methylthio, and Z is O or a bond,
in which the active ingredients (a) and (b) are present in each case in free form or in the form of a pharmaceutically acceptable salt, optionally, at least one pharmaceutically acceptable carrier and/or, optionally, a standard anti-diarrheal; for simultaneous, separate or sequential use.

2. Combination according to claim 1 comprising an epothilone derivative of formula I wherein A represents O, R is lower alkyl or hydrogen, R' is methyl and Z is O or a bond.

3. Combination according to claim 1 comprising an epothilone derivative of formula I wherein A represents O, R is lower alkyl or hydrogen, R' is methoxy, ethoxy, amino, methylamino, dimethylamino, aminomethyl or methylthio, and Z is O or a bond.

4. Combination according to any one of claims 1 to 3 which is a combined preparation or a pharmaceutical composition.

5. Combination according to any one of claims 1 to 4 wherein the antineoplastic antimetabolite is selected from 5-fluorouracil, tegafur, gemcitabine and capecitabine.

6. Combination according to claim 1 wherein the antiproliferatively active ingredients are (a) the antineoplastic antimetabolite gemcitabine and (b) the epothilone derivative of formula I wherein A represents O, R is methyl, R' is methyl and Z is O.

7. Combination according to claim 1 wherein the antiproliferatively active ingredients are (a) the antineoplastic antimetabolite capecitabine and (b) the epothilone derivative of formula I wherein A represents O, R is methyl, R' is methyl and Z is O.

8. Method of treating a warm-blooded animal having a proliferative disease comprising administering to the animal a combination according to any one of claims 1 to 7 in a quantity which is jointly therapeutically effective against a proliferative disease and in which the compounds can also be present in the form of their pharmaceutically acceptable salts.

9. A pharmaceutical composition comprising a quantity which is jointly therapeutically effective against a proliferative disease of a pharmaceutical combination according to any one of claims 1 to 7 and at least one pharmaceutically acceptable carrier.

10. Use of a combination according to any one of claims 1 to 7 for the treatment of a proliferative disease.

11. Use of a combination according to any one of claims 1 to 7 for the preparation of a medicament for the treatment of a proliferative disease.

12. Use of an antineoplastic antimetabolite in combination with an epothilone derivative of formula I 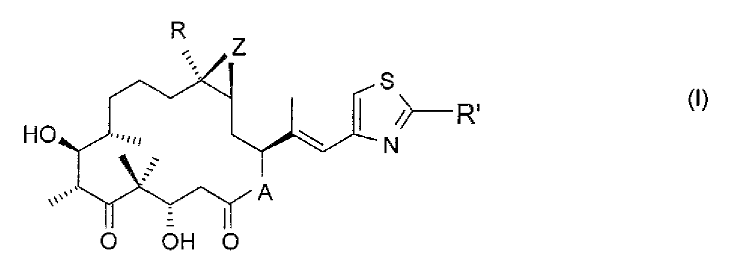 wherein A represents O or NR_{N}, wherein R_{N} is hydrogen or lower alkyl, R is hydrogen or lower alkyl, R' is methyl, methoxy, ethoxy, amino, methylamino, dimethylamino, aminomethyl or methylthio, and Z is O or a bond, for the preparation of a medicament for the treatment of a proliferative disease.

13. A commercial package comprising (a) an antineoplastic antimetabolite and (b) an epothilone derivative of formula I 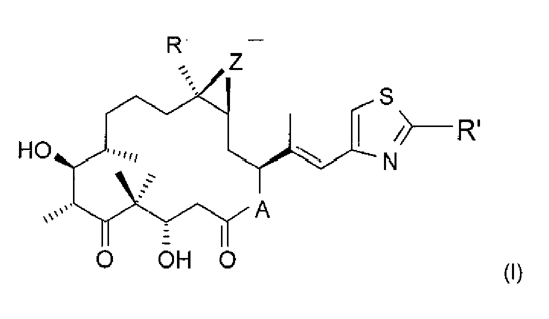 wherein A represents O or NR_{N}, wherein R_{N} is hydrogen or lower alkyl, R is hydrogen or lower alkyl, R' is methyl, methoxy, ethoxy, amino, methylamino, dimethylamino, aminomethyl or methylthio, and Z is O or a bond, and, optionally, a standard anti-diarrheal,
together with instructions for simultaneous, separate or sequential use thereof in the treatment of a proliferative disease.
